# EUROPÄISCHE PATENTSCHRIFT

(11) **EP 1 656 885 B1**
(45) Veröffentlichungstag und Bekanntmachung des Hinweises auf die Patenterteilung: **04.07.2007**
(21) Anmeldenummer: 05024511.7
(22) Anmeldetag: 10.11.2005
(51) Int. Cl.: A61B 5/16

(54) **Vorrichtung zur Durchführung eines neuropsychologischen Tests**
Device for performing a neuropsychological test
Appareil pour un test neuropsychologique

(30) Priorität: 11.11.2004 DE 102004054656
(43) Veröffentlichungstag der Anmeldung: 17.05.2006
(73) Patentinhaber: Wittling, Werner, Dr., 66540 Neunkirchen (DE); Wittling, Ralf Arne, 66540 Neunkirchen (DE)
(72) Erfinder: Wittling, Werner, Dr., 66540 Neunkirchen (DE); Wittling, Ralf Arne, 66540 Neunkirchen (DE)
(74) Vertreter: Becker, Bernd

(56) Entgegenhaltungen:
- US-A- 4 516 939
- US-A- 5 002 065

## Beschreibung

Die Erfindung bezieht sich auf eine Vorrichtung zur Durchführung eines neuropsychologischen Tests mit einem Schalter, der einen bewegbaren Stift umfasst, mit dem ein Reiz auf eine Fingerspitze einer zu untersuchenden Person ausübbar ist.

Zur systematischen Analyse des normalen oder durch eine Hirnschädigung beeinträchtigten sensu-motorischen Reaktionssystems von Personen mit Hilfe einer standardisierten und reproduzierbaren Messtechnik werden unterschiedliche Vorrichtungen verwendet. Mit quantifizierbaren und reproduzierbaren Messergebnissen, die mit den Vorrichtungen ermittelt werden, können Rückschlüsse auf die funktionale Effizienz, Integrität und Reaktivität des Gehirns bzw. bestimmter Hirnregionen, die in einem engen Bezug zu den untersuchten sensu-motorischen Hirnregionen stehen, gezogen werden. Dies sind insbesondere die Frontal- und Parietalregionen der beiden Hemisphären. Hieraus können gegenwärtige oder zukünftige Verhaltensweisen der getesteten Person erklärt oder vorhergesagt werden bzw. Rehabilitationsmaßnahmen zur Behebung einer Störung festgelegt werden.

Es werden normale, nicht hirngeschädigte Personen untersucht, um für jede Altersgruppe bzw. jedes Geschlecht durchschnittliche spezifische Leistungsmerkmale und Verhaltensaspekte zu ermitteln, wobei auch Einflüsse von Medikamenten, Alkohol oder dergleichen im Rahmen einer Leistungsmessung erfasst werden können. Im Weiteren kann bei einer vorliegenden Hirnschädigung, die z. B. durch einen Hirninfarkt, eine traumatische Hirnschädigung, einen Hirntumor oder eine degenerative Hirnveränderung, wie Morbus Alzheimer, verursacht wurde, das funktionale Potential der Person erfasst werden. Ebenso können zerebrale Korrelate psychiatrischer Erkrankungen, wie Schizophrenie oder Depression, festgestellt und entsprechende Rehabilitationsmaßnahmen festgelegt werden.

Die DE 196 33 866 A1 zeigt eine entsprechende Vorrichtung, bei der eine Person so schnell wie möglich für eine Zeitdauer von 15 Sekunden mit dem rechten und/oder linken Zeigefinger eine oder zwei Morsetasten so oft wie möglich drücken muss. Dabei ist der Morsetaste ein Drucksensor zugeordnet, um neben der Tasthäufigkeit auch den jeweiligen Tastdruck zu erfassen. Die Vorgabe eines bestimmten Tastrhythmus oder die Betätigung unterschiedlicher Morsetasten in Abhängigkeit eines Signals ist nicht offenbart.

Im Weiteren beschreibt die DE 41 42 248 A1 eine Tappingvorrichtung, die eine Vielzahl sich kreuzender, gegeneinander isolierter Leiterbahnen umfasst. Je nachdem an welche Leiterbahnen über einen Zufallsgenerator gesteuert eine Spannung angelegt wird, wird diese Kreuzungsstelle als vorgegebener Tappingort ausgewiesen. Dieser Tappingort muss von der Person, beispielsweise mit einer Fingerspitze, gedrückt werden, wobei das Drücken elektromagnetisch erfassbar ist. Ebenfalls kann der Druck, der von der Fingerspitze ausgeübt wird, erfasst werden. Die Tappingvorrichtung hat den Nachteil, dass sie relativ großflächig ausgeführt sein muss, um unterschiedliche Tappingorte auszuweisen.

Des Weiteren ist aus der US 2003/0055322 A1 eine neurologische Testvorrichtung bekannt, bei der beispielsweise über farbige LED's ein Signal an eine zu untersuchende Person übermittelt wird, die daraufhin u. a. einen berührungsempfindlichen Bildschirm berühren muss. Hierbei erfolgt die Informationsübermittlung vom Auge an das Gehirn, weshalb keine Informationsübermittlung von den Fingerspitzen zum Gehirn erfolgt.

Ferner beschreibt die US-A-4 516 939 ein Fingersteuerungssystem, bei dem jedem Finger ein Schalter zugeordnet ist, der jeweils einen bewegbaren Stift umfasst. Die Schalter können von einer gemeinsamen Steuerungseinrichtung angesteuert werden, um jeweils die Stifte derart zu bewegen, dass sie einen Reiz auf die Fingerspitzen der Person ausüben. Die Person wiederum kann, beispielsweise wenn durch einen der Stifte eine Reizung eines Fingers erfolgte, den Stift beaufschlagen, was von der Steuerungseinrichtung erfasst wird. Hierbei kann der Schalter allerdings nur dann betätigt werden, wenn der Stift zuvor in eine ausgefahrene Stellung verlagert wurde. Dies ist insofern nachteilig, als eine Fehlbedienung eines Schalters bei eingezogenem Stift mit der Vorrichtung nicht erfassbar ist, da der in einer eingezogenen Position angeordnete Stift nicht noch weiter eingedrückt werden kann.

Es ist Aufgabe der Erfindung, eine Vorrichtung zur Durchführung eines neuropsychologischen Tests der eingangs genannten Art zu schaffen, mit der in einfacher Weise eine neuropsychologische Untersuchung durchgeführt werden kann, wobei auch eine unabsichtliche Betätigung bzw. eine fehlerhafte Betätigung eines Schalters der Vorrichtung erfasst werden kann.

Erfindungsgemäß wird die Aufgabe dadurch gelöst, dass dem Schalter ein mit der Fingerspitze betätigbarer Druckaufnehmer zugeordnet ist, der unabhängig von der Position des Stiftes beaufschlagbar ist.

Der Kerngedanke der Erfindung besteht darin, dass die Reizung der Haut einer Fingerspitze der zu untersuchenden Person in an sich bekannter Weise über einen zwischen zwei Positionen bewegbaren Stift, vorzugsweise ein Metallstift, erfolgt. Hierbei wird die Fingerspitze beispielsweise durch die Verlagerung des Stifts von einer eingezogenen in eine ausgezogene Position gereizt. Des Weiteren umfasst der Schalter bzw. die gesamte Vorrichtung einen Druckaufnehmer, der dem Schalter bzw. dem Stift derart zugeordnet ist, dass er von der gleichen Fingerspitze, die von dem Stift gereizt wird, betätigbar ist. Dabei können der Stift und der Druckaufnehmer in an sich beliebiger Weise, bevorzugt aber wie im Folgenden beschrieben, angeordnet werden. Hierbei ist es für den Fachmann ersichtlich, dass der Vorrichtung bzw. dem Schalter eine Steuerung zugeordnet ist, um einerseits mit dem Schalter bzw. dem Stift einen oder mehrere Reize in einer gewünschten zeitlichen Abfolge auf die Fingerspitze auszuüben sowie eine Betätigung des Druckaufnehmers durch die Fingerspitze zu erfassen. Dabei ist die Steuerung vorzugsweise derart ausgelegt, dass die Messergebnisse, d. h. neben der Ansteuerung des Stifts die Betätigung des Druckaufnehmers, in Abhängigkeit von der Zeit aus der Steuerung ausgelesen und vorzugsweise an einem PC mit einem hierfür geeigneten Programm ausgewertet wird.

Der Vorteil der Erfindung liegt darin, dass eine Reaktion der zu untersuchenden Person jederzeit mit der Vorrichtung erfassbar ist, da der Druckaufnehmer unabhängig von der Position des Stifts von der Fingerspitze betätigt bzw. gedrückt werden kann, es also nicht erforderilch ist, den Stift nieder zu drücken, woraus auch eine verhältnismäßig kurze Reaktionszeit resultiert. Insbesondere ist es möglich, eine Beaufschlagung des Druckaufnehmers zu erfassen, auch wenn vorher der Stift die Fingerspitze nicht gereizt hat. Hierbei ist es nicht notwendig, die Fingerspitze oder sogar den ganzen Finger nach erfolgter Reizung an eine andere Position zu bewegen, um den Druckaufnehmer zu betätigen. Es versteht sich, dass die Reaktionszeiten, d. h. die Zeitspanne zwischen dem Reizen der Fingerspitze und dem Betätigen des Druckaufnehmers, mit der Vorrichtung erfasst werden können. Ebenso ist es möglich, Fehlerquoten, d. h. eine fehlerhafte Betätigung des Druckaufnehmers, sowie deren zeitliche Verläufe zu erfassen, da bei zunehmender Testdauer üblicherweise eine Ermüdung der Person und damit einhergehend eine Erhöhung der Fehlerquote auftritt.

Bevorzugt ist der Druckaufnehmer in Form einer Tastplatte ausgebildet und der Stift erstreckt sich durch eine Ausnehmung der Tastplatte. Die Tastplatte ist in axialer Richtung des Stifts, in der dieser auch zur Reizung der Fingerspitze bewegt wird, niederdrückbar, um durch dieses Niederdrücken die Reaktion der Person auf die Reizung durch den Stift zu erfassen. Die Tastplatte kann als runde Scheibe aus Metall und/oder Kunststoff mit einer mittigen Ausnehmung für den Stift ausgeformt sein, wobei der Durchmesser der Tastplatte ungefähr der Größe einer Fingerspitze entspricht.

In Ausgestaltung ist der Druck, mit dem der Druckaufnehmer von der Fingerspitze betätigt wird, erfassbar, wobei die Größe des Drucks beispielsweise über ein der Tastplatte zugeordnetes Piezoelement gemessen werden kann. Somit kann die Kraft, die von der Fingerspitze auf die Tastplatte ausgeübt wird, auch im zeitlichen Verlauf des Niederdrückens erfasst werden, um hieraus weitere sensu-motorische Informationen ableiten zu können.

Zur Ermöglichung einer umfassenden Untersuchung der Person umfasst die Vorrichtung vier oder fünf Schalter mit einem bewegbaren Stift, dem jeweils ein Druckaufnehmer zugeordnet ist, wobei die Schalter ergonomisch angeordnet sind, damit bei einer entspannten Handhaltung der Person die Fingerspitzen jeweils auf den Schaltern zu liegen kommen. Beispielsweise können die Schalter an einer Oberseite eines Gehäuses der Vorrichtung angeordnet sein, um die Schalter bequem mit den Fingerspitzen betätigen zu können. Die Schalter können dabei bündig mit dieser Oberfläche ausgeführt sein oder leicht über die Oberfläche vorstehen. Es versteht sich, dass die Vorrichtung vorzugsweise aus zwei im Wesentlichen spiegelsymmetrisch aufgebauten gleichartigen Gehäusen mit Schaltern aufgebaut ist, um die Fingerspitzen beider Hände der Person zu reizen und hieraus sensu-motorische Rückschlüsse ableiten zu können. Dazu werden die in zwei getrennten Gehäusen angeordneten acht oder zehn Schalter selbstverständlich von einer gemeinsamen Steuerung angesteuert, um beispielsweise durch eine Reizung der Fingerspitzen der rechten Hand Aussagen über die Fähigkeiten der linken Gehirnhälfte zu erhalten und umgekehrt.

Zur Betätigung des Stifts, bzw. um diesen von einer ersten Position in eine zweite Position zu verlagern, wobei der Stift in dieser zweiten Position die Fingerspitze der zu untersuchenden Person reizt, dient ein Elektromagnet, der beispielsweise über ein Relais angesteuert wird, wobei der Stift bei einer Aktivierung des Elektromagneten durch das Magnetfeld ausgezogen wird.

Zur Erhöhung der Haltbarkeit der Vorrichtung bzw. der ihr zugeordneten Schalter ist die Tastplatte durch den Druck einer Fingerspitze gemeinsam mit dem Elektromagneten bewegbar, wobei die Bewegung parallel zur axialen Bewegungsrichtung des Stifts erfolgt. Hierfür sind die Tastplatte und der Elektromagnet in einer entsprechend ausgelegten Führung gelagert. Nach dem Erreichen einer Endlage wird die von der Fingerspitze ausgeübte Kraft von der Führung und/oder dem Gehäuse aufgenommen, weshalb ein zerstörender bzw. beschädigender Überhub ausgeschlossen ist.

Um den Schalter der Vorrichtung, nachdem der Druckaufnehmer bzw. die Tastplatte nicht mehr von einer Fingerspitze gedrückt wird, wieder in seine Ausgangsposition zurückzuverlagern, ist der Druckaufnehmer sowie gegebenenfalls der Elektromagnet gemeinsam von einer vorzugsweise als Mikrotasterfeder ausgelegten Feder mit einer Rückstellkraft beaufschlagt, um den Druckaufnehmer beispielsweise bei einem obenseitig an einem Gehäuse angeordneten Schalter wieder nach oben zu bewegen.

Für die Erfassung weiterer Verarbeitungsprozesse im Gehirn der Person sind der Vorrichtung weitere Signalmittel zugeordnet, um optische und/oder akustische Signale oder Reize zu übertragen. Dies können beispielsweise Leuchtmittel sein, um unterschiedliche Lichtsignale zu erzeugen, oder ein Lautsprecher, um Sprachmitteilungen, Töne oder dgl. auszugeben. Beispielsweise kann die Sprachmitteilung ausgegeben werden, dass trotz der Reizung eines Fingers keine Reaktion erfolgen soll. Hierdurch können Rückschlüsse auf die Verarbeitung dieser Signale im Gehirn bzw. auf die Weiterleitung der entsprechenden Befehle im Gehirn gezogen werden, insbesondere aus der hier auftretenden Fehlerquote.

Zur Durchführung eines neuropsychologischen Tests kann die Vorrichtung in unterschiedlichen Betriebsarten angesteuert werden. Beispielsweise kann ein Druckaufnehmer mit der Fingerkuppe so oft wie möglich innerhalb eines vorgegebenen Zeitraums oder entsprechend eines durch den Stift vorgegebenen Rhythmus betätigt werden. Im Weiteren ist auch eine Dauerreizung, beispielsweise durch Vibration des Striftes, zur Aufmerksamkeitsuntersuchung möglich. Hierbei kann entweder nur ein einzelner Finger oder mehrere Finger aufeinander folgend erfasst werden. Vorzugsweise erfolgt immer ein Seitenvergleich der identischen Finger beider Hände. Dabei können die Anzahl der Taps, d. h. ein Drücken, für die vorgegebene Gesamtdauer, die Anzahl der Taps pro Zeitabschnitt zur Bestimmung einer Verlaufskurve, der zeitliche Abstand zweier aufeinander folgender Taps, der zeitliche Verlauf sowie der Rhythmus und eine Fehlerquote erfasst werden, um, beispielsweise bei gleichzeitiger Erfassung der Taps zweier Hände, die manuelle Koordination, die Rhythmizität und Stabilität der Fingermotorik sowie die motorische Ermüdung im zeitlichen Verlauf zu messen und die beiden Gehirnhälften miteinander zu vergleichen sowie eine eventuell vorliegende Hirnfunktionsstörung lokalisieren zu können. Es versteht sich, dass gleichzeitig auch ein EEG des Patienten aufgenommen werden kann. Hierzu ist die Vorrichtung mit einer Schittstelle versehen, über die die Aktivierung des Stiftes bzw. ein Signal des Druckaufnehmers an das EEG weitergeleitet wird.

Ebenso kann für einen oder mehrere Finger beider Hände die Reaktionszeit erfasst werden, in dem die Zeitspanne zwischen der Ausübung des Reizes durch den sich bewegenden Stift und die Betätigung des Druckaufnehmers erfasst wird. Zusätzlich kann, beispielsweise über ein Lichtsignal, dem Patienten angezeigt werden, dass er, obwohl eine Reizung erfolgte, den Druckaufnehmer nicht betätigen soll, um derart eine so genannte Wahlreaktionszeit auszumessen. Ebenso ist es möglich, beispielsweise einen Finger der linken Hand zu reizen und von der Person zu verlangen, mit dem entsprechenden Finger der rechten Hand den Druckaufnehmer zu betätigen und umgekehrt. Hiermit können interhemisphärische Übertragungsprozesse ausgemessen werden, um auf eine Hirnfunktionsstörung, die das interhemisphärische Verbindungssystem betrifft, zu erfassen.

Mit der Vorrichtung können die motorischen und taktilen Leistungsmerkmale einzelner Finger isoliert und differenziert erfasst werden. Prinzipiell kann mit der Vorrichtung auch die Finger- und Handmotorik einer Person, insbesondere nach einer Hirnschädigung, trainiert werden.

Es versteht sich, dass vorzugsweise standardisierte Reize, z. B. in einem zeitlich festen Verlauf, ausgeübt werden, auf die der Patient reagieren soll. Sonach können, sobald von einer ausreichenden Anzahl gesunder Personen ein statistisch zuverlässiger Mittelwert vorliegt, die Werte der zu untersuchenden Personen mit diesem Mittelwert verglichen und hieraus beispielsweise auf krankheitsbedingte Abweichungen geschlossen werden.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispieles unter Bezugnahme auf die zugehörigen Zeichnungen näher erläutert. Es zeigt:

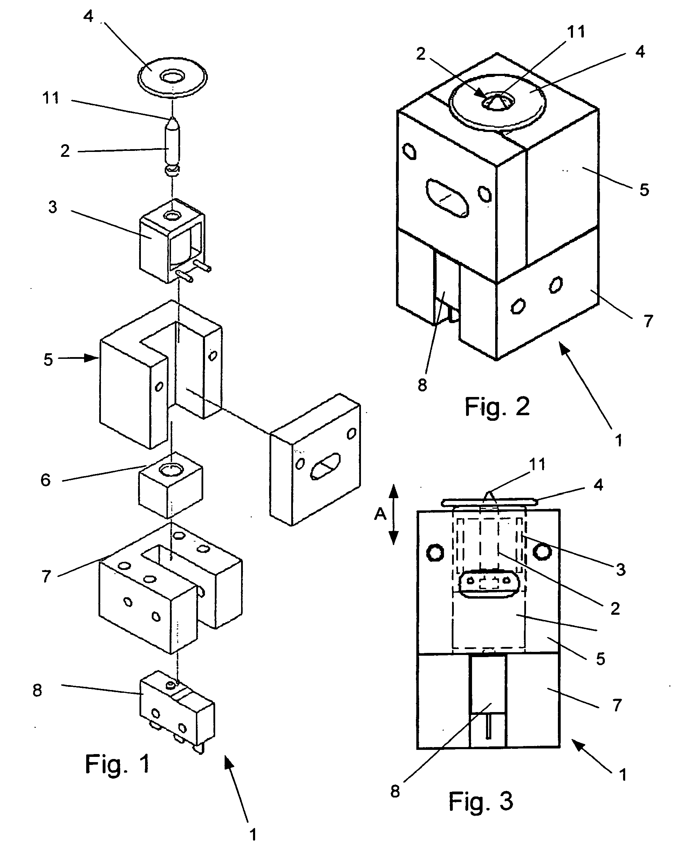

- Fig.1: eine Explosionsdarstellung eines Schalters der erfindungsgemäßen Vorrichtung,
- Fig.2: eine perspektivische Darstellung des Schalters nach Fig. 1,
- Fig.3: eine Seitenansicht des Schalters nach Fig. 1,

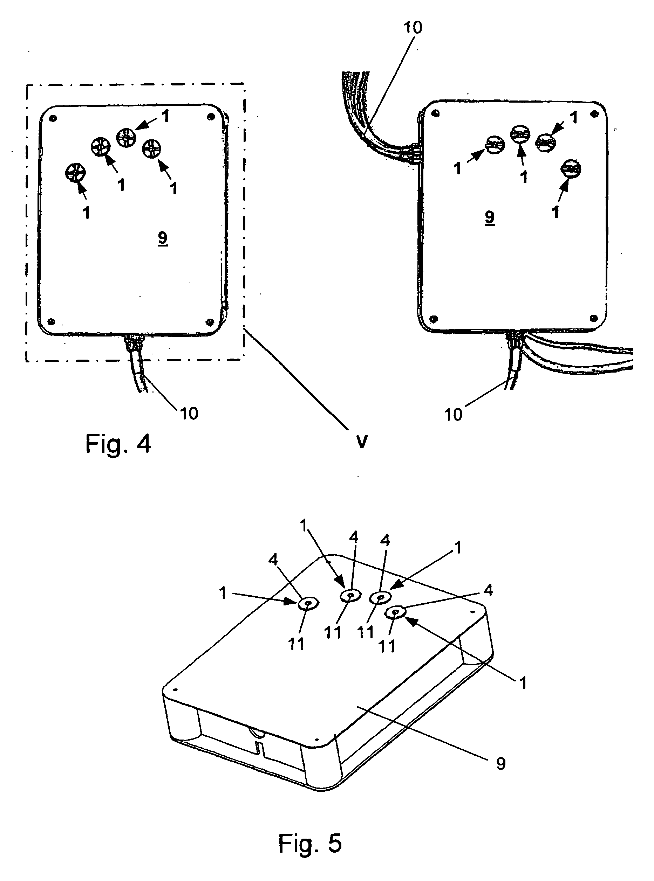

- Fig.4: zwei spiegelsymmetrische Gehäuse der Vorrichtung,
- Fig.5: eine perspektivische Darstellung der Einzelheit V nach Fig. 4,

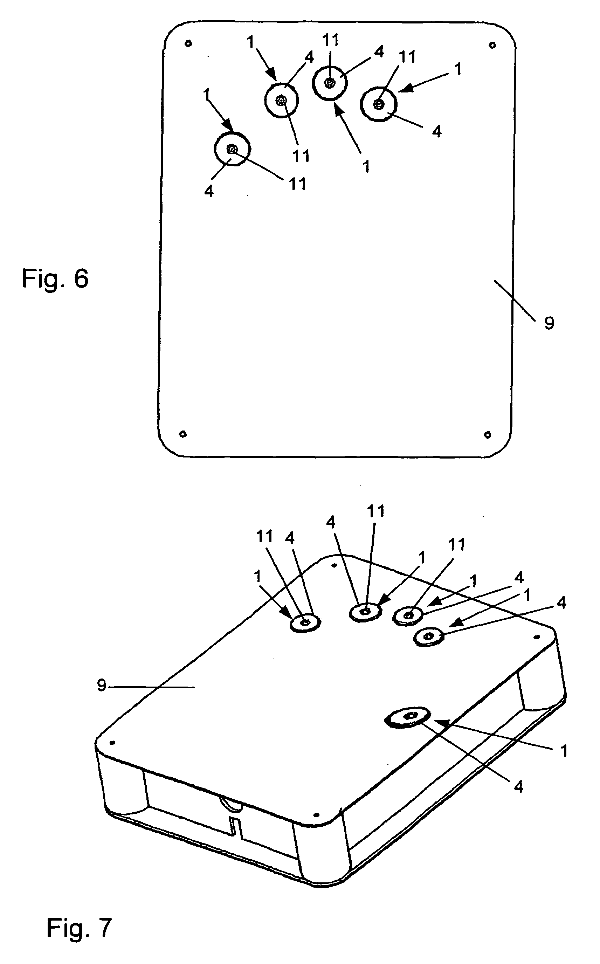

- Fig.6: eine Draufsicht auf die Darstellung nach Fig. 5,
- Fig.7: eine perspektivische Darstellung der Einzelheit V nach Fig. 4 in alternativer Ausgestaltung.

Der Schalter 1 besteht im Wesentlichen aus einem metallischen, mit einer endseitigen Spitze 11 versehenen Stift 2, der innerhalb eines Elektromagneten 3 angeordnet ist, wobei der Elektromagnet 3 derart ansteuerbar ist, dass der Stift 2 in seiner axialen Richtung von einer eingezogenen in eine ausgezogene Position verlagerbar ist. In dieser ausgezogenen Positionen steht der Stift 2 senkrecht nach oben über den Schalter 1 über und reizt mit seiner Spitze 11 die Haut einer auf dem Schalter 1 aufliegenden Fingerspitze einer zu untersuchenden Person.

Des Weiteren ist dem Schalter 1 ein Druckaufnehmer zugeordnet, der hier in Form einer ringförmigen Tastplatte 4 mit einer mittigen Ausnehmung für den Stift 2 ausgebildet ist. Wird der Druckaufnehmer bzw. die Tastplatte 4 betätigt, wird die Tastplatte 4 gemeinsam mit dem Stift 2 und dem Elektromagneten 3 in axialer Richtung, wie durch den Doppelpfeil A angedeutet, nach unten verlagert. Hierfür ist der Elektromagnet 3 in einer hier zweiteilig ausgebildeten Führung 5 verschiebbar angeordnet. Die Tastplatte 4 kann unabhängig von der Stellung des Stifts 2 betätigt werden.

Unterhalb des Elektromagneten 3 weist der Schalter 1 einen Beschleunigungsblock 6 auf, um die Bewegung des Elektromagneten 3 bzw. der Tastplatte 4 auf einen in einer Halterung 7 angeordneten Mikroschalter 8 zu übertragen. Mit dem Beschleunigungsblock 6 kann eine Bewegung des Elektromagneten 3 und der Tastplatte 4 gedämpft werden. Des Weiteren ermöglicht der Beschleunigungsblock 6 in Abhängigkeit von seiner Ausgestaltung eine Variation der Endgeschwindigkeit des Stiftes 2 und damit des ausgelösten Impulses. Zusätzlich weist der Mikroschalter 8 eine Feder auf, um nach dem Loslassen der Tastplatte 4 diese sowie den Elektromagneten 3 wieder nach oben in die ursprüngliche Position zurückzuverlagern. Die Führung 5 und die Halterung 7 bestehen vorzugsweise aus Metall und/oder Kunststoff.

Die Signale des Mikroschalters 8 werden über eine geeignete Steuerung, die hard- und/oder softwaremäßig implementiert ist, ausgelesen und der Elektromagnet 3 mittels der Steuerung in gewünschter Weise angesteuert, um unterschiedliche neuropsychologische Tests mit der Vorrichtung durchzuführen. Dazulässt sich die Steuerung derart variieren, dass Einzelimpulse, Mehrfachimpuse bis hin zu Dauerimpulsen (Vibration) des Stiftes 2 möglich sind. Der Impuls des Stiftes 2 kann auch durch eine Anpassung der Anschaltdauer bzw. eine Veränderung der Betriebsspannung des Elektromagneten 3 in einem weiten Bereich bis hinab zur Tastschwelle beeinflusst werden.

Die Vorrichtung besteht im Wesentlichen aus zwei spiegelsymmetrisch ausgebildeten Gehäusen 9, an deren Oberseiten jeweils mehrere Schalter 1 in ergonomischer Weise verteilt angeordnet sind, um jeweils vier Finger einer Hand zu stimulieren und die Reaktionen hierauf zu erfassen. Prinzipiell kann in den Gehäusen 9 jeweils auch ein fünfter Schalter 1 für den Daumen vorgesehen sein. Aus den als Handauflage dienenden Gehäusen 9 sind Leitungen 10 zur Energieversorgung sowie zur Ansteuerung der Schalter 1 bzw. zum Auslesen der Messdaten herausgeführt, wobei die Auswertung und Ansteuerung vorzugsweise mit einem entsprechenden Programm über einen PC erfolgt.

## Patentansprüche

1. Vorrichtung zur Durchführung eines neuropsychologischen Tests mit einem Schalter (1), der einen bewegbaren Stift (2) umfasst, mit dem ein Reiz auf eine Fingerspitze einer zu untersuchenden Person ausübbar ist, **dadurch gekennzeichnet, dass** dem Schalter (1) ein mit der Fingerspitze betätigbarer Druckaufnehmer zugeordnet ist, der unabhängig von der Position des Stiftes (2) beaufschlagbar ist.

2. Vorrichtung nach Anspruch 1, **dadurch gekennzeichnet, dass** der Druckaufnehmer eine Tastplatte (4) umfasst und der Stift (2) sich durch eine Ausnehmung der Tastplatte (4) erstreckt.

3. Vorrichtung nach Anspruch 1 oder 2, **dadurch gekennzeichnet, dass** der Druck, mit dem der Druckaufnehmer betätigt wird, messbar ist, insbesondere über ein Piezoelement.

4. Vorrichtung nach einem der Ansprüche 1 bis 3, **dadurch gekennzeichnet, dass** zwei Gehäuse (9) mit jeweils vier oder fünf ergonomisch angeordneten Schaltern (1) mit Stiften (2) und Druckaufnehmern vorgesehen sind.

5. Vorrichtung nach einem der Ansprüche 1 bis 4, **dadurch gekennzeichnet, dass** der Stift (2) mit einem Elektromagneten (3) betätigbar ist.

6. Vorrichtung nach Anspruch 5, **dadurch gekennzeichnet, dass** der Druckaufnehmer gemeinsam mit dem Elektromagneten (3) bewegbar ist.

7. Vorrichtung nach Anspruch 5 oder 6, **dadurch gekennzeichnet, dass** der Druckaufnehmer und der Elektromagnet (3) von einer Federkraft beaufschlagt sind.

8. Vorrichtung nach einem der Ansprüche 1 bis 7, **dadurch gekennzeichnet, dass** weitere, insbesondere optische und/oder akustische, Signalmittel vorgesehen sind.

## Claims

1. Apparatus for accomplishing a neuropsychological test with a switch (1), which includes a displaceable pin (2) whereby a stimulus can be exerted upon a finger-tip of a person to be tested, **characterised in that** a pressure sensor, which is actuatable with the finger-tip and can be acted-upon independently of the position of the pin (2), is associated with the switch (1).

2. Apparatus according to claim 1, **characterised in that** the pressure sensor includes a scanning plate (4), and the pin (2) extends through a recess in the scanning plate (4).

3. Apparatus according to claim 1 or 2, **characterised in that** the pressure, whereby the pressure sensor is actuated, is measurable, more especially via a piezoelectric element.

4. Apparatus according to one of claims 1 to 3, **characterised in that** two housings (9) are provided, each with four or five ergonomically disposed switches (1) having pins (2) and pressure sensors.

5. Apparatus according to one of claims 1 to 4, **characterised in that** the pin (2) is actuatable with an electromagnet (3).

6. Apparatus according to claim 5, **characterised in that** the pressure sensor is displaceable jointly with the electromagnet (3).

7. Apparatus according to claim 5 or 6, **characterised in that** the pressure sensor and the electromagnet (3) are acted-upon by a resilient force.

8. Apparatus according to one of claims 1 to 7, **characterised in that** additional, more especially visual and/or audible, signalling means are provided.

## Revendications

1. Dispositif pour réaliser un test neuropsychologique avec un commutateur (1) qui comprend une goupille mobile (2) avec laquelle un stimulus peut être exercé sur le bout d'un doigt d'une personne à examiner, **caractérisé en ce qu'**un capteur de pression pouvant être actionné à l'aide du bout de doigt est associé au commutateur (1), ledit capteur de pression pouvant être alimenté indépendamment de la position de la goupille (2).

2. Dispositif selon la revendication 1, **caractérisé en ce que** le capteur de pression comprend une plaque de contact (4) et la goupille (2) s'étend à travers un évidement de la plaque de contact (4).

3. Dispositif selon la revendication 1 ou 2, **caractérisé en ce que** la pression à l'aide de laquelle le capteur de pression est actionné, peut être mesurée, plus particulièrement via un piézoélément.

4. Dispositif selon l'une quelconque des revendications 1 à 3, **caractérisé en ce que** deux boîtiers (9) comportant chacun quatre ou cinq commutateurs (1) disposés ergonomiquement, avec des goupilles (2) et des capteurs de pression sont prévus.

5. Dispositif selon l'une quelconque des revendications 1 à 4, **caractérisé en ce que** la goupille (2) peut être actionnée à l'aide d'un électroaimant (3).

6. Dispositif selon la revendication 5, **caractérisé en ce que** le capteur de pression peut être déplacé ensemble avec l'électroaimant (3).

7. Dispositif selon la revendication 5 ou 6, **caractérisé en ce que** le capteur de pression et l'électroaimant (3) sont sollicités par une tension de ressort.

8. Dispositif selon l'une quelconque des revendications 1 à 7, **caractérisé en ce que** d'autres moyens de signalisation, plus particulièrement optiques et/ou acoustiques, sont prévus.
